# EUROPEAN PATENT APPLICATION

(11) **EP 2 431 012 A1**
(43) Date of publication of application: **21.03.2012**
(21) Application number: 10774968.1
(22) Date of filing: 13.05.2010
(51) Int. Cl.: A61F 13/15, A61F 13/472, B65D 75/20

(54) **PACKAGED ABSORBENT ARTICLE**

(30) Priority: 14.05.2009 JP 2009118102
(71) Applicant: Unicharm Corporation, Ehime 799-0111 (JP)
(72) Inventor: KATO, Nobuyuki, Kanonji-shi Kagawa 769-1602 (JP); KASHIWAGI, Masahiro, Kanonji-shi Kagawa 769-1602 (JP); TAMURA, Tatsuya, Kanonji-shi Kagawa 769-1602 (JP); NAKAYAMA, Nahomi, Kanonji-shi Kagawa 769-1602 (JP)
(74) Representative: Peter, Julian
(86) International application number: PCT/JP2010/058114
(87) International publication number: WO 2010/131711

(57) **Abstract**

While a protection sheet 60 is disposed on a non-skin-surface 20a of an absorbent article 1, part of the protection sheet 60 is folded back in such a manner as to reach a position where the part overlaps other part, which comes in contact with the non-skin surface 20a of the absorbent article 1. An end portion 60a of the protection sheet 60 is bonded, while being folded back, to the protection sheet 60 by means of an adhesive material 70. Edge portions 60b and 60c on both sides of the protection sheet 60 in a longitudinal direction of the protection sheet 60 are bonded together through thermal fusion bonding while being folded. A second adhesive region 71 coated with the adhesive material 70 is formed in a predetermined position which faces the non-skin-surface 20a of a backsheet 20. The second adhesive region 71 is formed at a position corresponding to a non-adhesive region 52.

## Description

### [Technical Field]

The present invention relates to a wrapped absorbent article which includes an adhesive material provided on a part of a main body and configured to attach the main body to a clothing item, and which is individually wrapped with a protection sheet.

### [Background Art]

Absorbent articles, such as sanitary napkins, are commonly sold in individual wrappings. Since some of such absorbent articles have an adhesive material applied thereon, it is necessary to prevent the adhesive material from sticking to a wrapping material in a process of wrapping the absorbent articles individually. Conventionally, a method has been known for individually wrapping an absorbent article by using a protection sheet which protects an adhesive material (for example, refer to Patent Document 1). In the absorbent article, the protection sheet also functions as a wrapping material.

A surface of the protection sheet is treated so as to make the adhesive material separable from the surface without reducing an adhesive force of the adhesive material. Additionally, the protection sheet includes either a part coated with an adhesive material or an adhesive tape provided on an end portion of the protection sheet. Then, the adhesive material (or the adhesive tape) is attached to a part of the protection sheet while the absorbent article is in a folded state. Thereby, the absorbent article is wrapped by use of the protection sheet.

However, the above described conventional absorbent article has the following problems. Specifically, due to changes over time, in temperature, or the like, the adhesive material on the adhesive tape in some cases permeates the protection sheet and then penetrates into the adhesive material applied on the absorbent article. In such cases, these adhesive materials are dissolved with each other to generate an adhesive force stronger than expected. Such strong adhesive force may make it difficult to unwrap the absorbent article, or to peel off the protection sheet from the absorbent article because of the protection sheet having been broken at the time of unwrapping.

### [Prior Art Document]

### [Patent Document]

[Patent Document 1] Japanese Patent Application Publication No. Heisei 8-324635 (p. 4, FIGs. 5 and 8, and so on)

### [Summary of Invention]

A wrapped absorbent article includes an absorbent article including a top sheet including a contact region which comes in contact with an excretory part of a wearer, a backsheet having liquid impermeability which allows no permeation of liquid, and including a first adhesive material which is provided on a non-skin-surface of the backsheet to be attached to a clothing item, and an absorber disposed between the topsheet and the backsheet; and a protection sheet having a facing surface which faces the absorbent article on the backsheet side while keeping the first adhesive material separable. The absorbent article is wrapped with the protection sheet in a state where the non-skin-surface of the absorbent article and the facing surface of the protection sheet are in contact with each other. The non-skin-surface of the backsheet is provided with a first adhesive region coated with the first adhesive material and a non-adhesive region not coated with the first adhesive material. The protection sheet includes a second adhesive region coated with a second adhesive material. The absorbent article and the protection sheet are bent with the topsheet inside so that the second adhesive region overlaps a region included in the non-adhesive region. The protection sheet is attached to the region included in the non-adhesive region of the protection sheet at the second adhesive region.

### [Brief Description of Drawings]

[FIG. 1] FIG. 1 is a plan view of a top surface side of an absorbent article according to an embodiment.
[FIG. 2] FIG. 2 is a plan view of a back surface side of the absorbent article according to the embodiment.
[FIG. 3] FIG. 3 is a schematic view describing how the absorbent article is folded and wrapped with a protection sheet.
[FIG. 4] FIG. 4 is a perspective view showing a wrapped absorbent article, with a part of the wrapped absorbent article cut away, according to the embodiment.
[FIG. 5] FIG. 5 is a cross-sectional view taken along an A-A line of the wrapped absorbent article shown in FIG. 4.
[FIG. 6] FIG. 6 is a perspective view showing a wrapped absorbent article, with a part of the wrapped absorbent article cut away, shown as a modified example.
[FIG. 7] FIG. 7 is a cross-sectional view taken along a B-B line of the wrapped absorbent article shown in FIG. 6.
[FIG. 8] FIG. 8 is a perspective view showing a wrapped absorbent article, with a part of the wrapped absorbent article cut away, shown as a modified example.
[FIG. 9] FIG. 9 is a cross-sectional view taken along a C-C line of the wrapped absorbent article shown in FIG. 8.
[FIG. 10] FIG. 10 is a perspective view showing a wrapped absorbent article, with a part of the wrapped absorbent article cut away, shown as a modified example.
[FIG. 11] FIG. 11 is a cross-sectional view taken along a D-D line of the wrapped absorbent article shown in FIG. 10.
[FIG. 12] FIGs. 12 (a) to 12 (d) are plan views each describing a modified example of a first adhesive region and a non-adhesive region which are formed on a backsheet.

### [Description of Embodiments]

A wrapped absorbent article according to the embodiment will be described with reference to the accompanying drawings. The wrapped absorbent article is obtained by wrapping an absorbent article with a protection sheet.

Note that, in the following description of the drawings, same or similar reference signs denote same or similar elements and portions. In addition, it should be noted that the drawings are schematic and ratios of dimensions and the like are different from actual ones. Therefore, specific dimensions and the like should be determined in consideration of the following description. Moreover, the drawings also include portions having different dimensional relationships and ratios from each other.

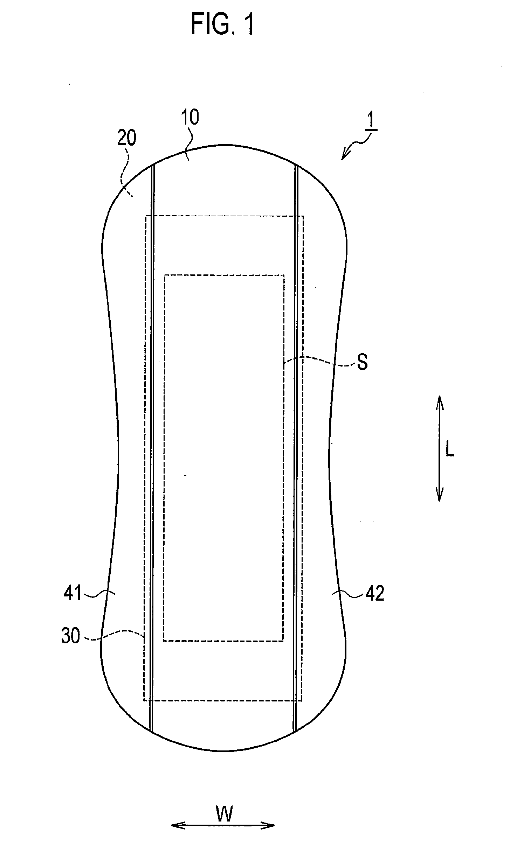

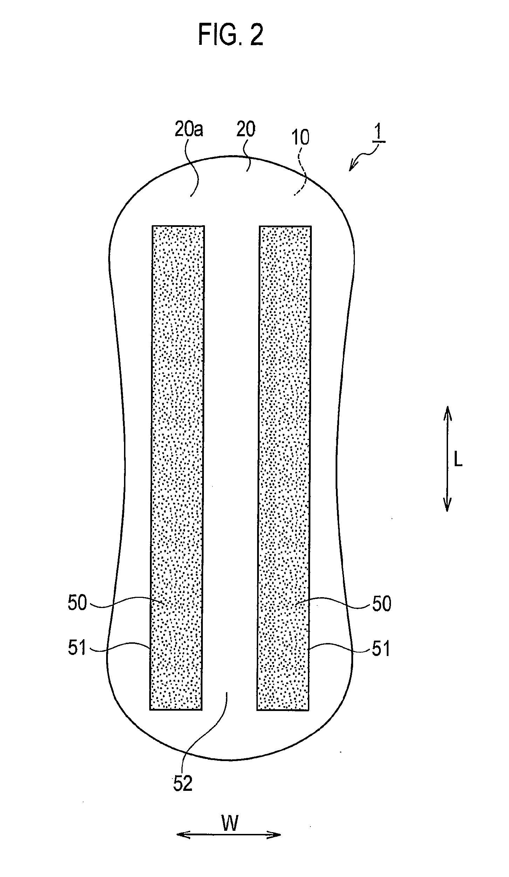

In the first place, a description will be provided to an absorbent article 1 which is included in a wrapped absorbent article 100 according to the embodiment. FIG. 1 is a plan view of a top surface side of the absorbent article 1 according to the embodiment. FIG. 2 is a plan view of a back surface side of the absorbent article 1.

The absorbent article 1 is, for example, a sanitary napkin. The absorbent article 1 includes: a topsheet 10 which comes in contact with the skin of a wearer; a liquid-impermeable backsheet 20 which does not allow permeation of liquid; and an absorber 30. The absorber 30 is disposed between the topsheet 10 and the backsheet 20. Accordingly, the absorber 30 is indicated by a broken line in FIG. 1. The absorber 30 is disposed in a central portion of the absorbent article 1 in a longitudinal direction L of the absorbent article 1. Additionally, the absorbent article 1 includes sidesheets 41 and 42 each provided to an outer side of the absorber 30 in a width direction W of the absorbent article 1 perpendicular to the longitudinal direction L of the absorbent article 1.

The topsheet 10 includes a region S which comes in contact with a vaginal opening of a wearer. The topsheet 10 has a substantially same length as the length of the backsheet 20. The shape of an end portion of the topsheet 10 in a longitudinal direction of the topsheet 10 is substantially same as the shape of an end portion of the backsheet 20 in a longitudinal direction of the backsheet 20. The topsheet 10 covers at least a surface of the absorber 30.

The material for the topsheet 10 is not particularly limited as long as a sheet-like material, such as a nonwoven cloth, a woven cloth, a perforated plastic sheet, and a mash sheet, which has a structure allowing permeation of liquid, is adopted. As a raw material for the nonwoven or woven cloth, any of natural fiber and chemical fiber is adoptable.

Examples of the natural fiber include cellulose, such as crushed pulp and cotton. Examples of the chemical fiber include: regenerated cellulose, such as rayon and fibril rayon; semi-synthetic cellulose, such as acetate and triacetate; a thermoplastic hydrophobic chemical fiber; and a thermoplastic hydrophobic chemical fiber treated with a hydrophilization treatment. Examples of the thermoplastic hydrophobic chemical fiber include: monofilament of polyethylene (PE), polypropylene (PP), polyethylene terephthalate (PET) or the like; and fiber obtained through graft polymerization of polyethylene and polypropylene, and a composite fiber having a core-in-sheath structure or the like.

As a method for producing the unwoven cloth, any one of dry processes (such as carding, spun-bonding, melt- blowing, and air-laying processes) and a wet process is adoptable. Alternatively, a combination of multiple ones of the dry and wet processes is also adoptable. Other adoptable methods include thermal bonding, needle punching, and chemical bonding. The method for producing the unwoven cloth is not limited to the above described methods.

A spun lace formed into a sheet by a water-flow entangling method may alternatively be used as the topsheet 10. Further, it is also possible to adopt as the topsheet 10 a non-woven cloth having undulations on an upper layer side of the non-woven cloth or an undulated non-woven cloth having uneven weight per unit area by the application of an air blowing process onto a nonwoven cloth in a web formation.. By forming the undulations on the surface, it is possible to reduce the diffusion of body fluid along the surface of the topsheet 10 before the body fluid permeates the topsheet 10.

As for the backsheet 20, a film formed mainly of polyethylene, polypropylene, or the like, an air-permeable resin film, a sheet formed by joining an air-permeable resin film to a nonwoven cloth, such as a spunbond and a spanlace, is adoptable. It is preferable that the backsheet 20 be made of a material flexible enough not to bring discomfort when the absorbent article 1 is worn.

The absorber 30 includes a hydrophilic fiber and pulp. For example, the following materials may be used by itself or in combination with others: cellulose, such as crushed pulp and cotton; regenerated cellulose, such as rayon and fibril rayon; semi-synthetic cellulose, such as acetate and triacetate; a granular polymer; a fibrous polymer; a thermoplastic hydrophobic chemical fiber; and a thermoplastic hydrophobic chemical fiber treated with a hydrophilization treatment. Among these materials, it is preferable to adopt crushed pulp in consideration of low cost and easiness of the formation of an absorber. A mixture obtained by mixing a polymer absorber into a hydrophilic fiber may be used alternatively as the absorber 30. In this embodiment, the polymer absorber is made of a granular polymer having absorbability and hygroscopicity, such as a sodium acrylate copolymer. The absorber 30 may be an air-laid sheet obtained by forming hydrophilic fiber or powder into a sheet in an air-laying process.

The material of the sidesheets 41 and 42 may be selected from materials similar to those of the topsheet 10. However, it is preferable that the material be hydrophobic or water-repellent, for the purpose of preventing menstrual blood to flow to the outside of the absorbent article 1 beyond the sidesheets 41 and 42. Specifically, a spun-bonded unwoven cloth and an SMS unwoven cloth are listed as examples. The sidesheets 41 and 42 constitute a contact surface which comes in contact with the skin. For this reason, it is preferable that a breathable unwoven cloth be used for the purpose of reducing skin irritation caused by abrasion. The sidesheets 41 and 42 are arranged to both sides of the topsheet 10.

In the absorbent article 1, peripheries of the topsheet 1, sidesheets 41 and 42, and backsheet 20 are bonded together, and thereby the absorber 30 is included inside. As a method for joining the topsheet 10 and the backsheet 20, any one or combination of a heat-embossing process, ultrasound, and a hot-melt adhesive is adoptable.

As shown in FIG. 2, on a surface, which attaches to a clothing item (referred to as a non-skin-surface 20a), of the backsheet 20, first adhesive regions 51 coated with an adhesive material 50 which adheres to clothing item and a non-adhesive region 52 not coated with adhesive material 50 thereon are formed. The first adhesive regions 51 are respectively formed in both side portions of the absorbent article 1 in a width direction of the absorbent article 1, extending continuously in the longitudinal direction of the absorbent article 1. The non-adhesive region 52 is formed continuously in the longitudinal direction of the absorbent article 1 in a central region including a region, corresponding to the region S of the topsheet 10, located on the backsheet 20.

In a case where the absorbent article 1 is conveyed along a longitudinal direction of the absorbent article 1 in a manufacturing process, the above described configurations of the first adhesive regions 51 and the non-adhesive region 52 are highly effective in manufacturing of the absorbent article 1.

Examples of the adhesive material 50 include: a styrene-based block polymer which is a thermoplastic resin; and a paraffin-based oil which is a plastic resin. Meanwhile, as examples of a tackifier resin for providing an adhesive force to the styrene-based block polymer, a synthetic resin and a natural resin are listed. Examples of the styrene-based block polymer include a styrene-ethylene-butadiene-styrene block copolymer (SEBS), a styrene-butadiene-styrene block copolymer (SBS), a styrene-isoprene-styrene block copolymer (SIS), and a styrene-ethylene-propylene-styrene block copolymer (SEPS). It is preferable that the molecular weight of a styrene part of the styrene-based block polymer be between 5000 and 20000. It is preferable to use a styrene-based block polymer in which the molecular weight of a styrene part accounts for 10% to 35% of the molecular weight of the entirety including a rubber part.

Examples of the synthetic resin used as the tackifier resin include an aliphatic petroleum resin, an aromatic petroleum resin, a copolymerized petroleum resin, a hydrogenated petroleum resin, a DCPD petroleum resin, and a pure-monomer petroleum resin. Meanwhile, examples of the natural resin include: a terpene-based resin which is a copolymer of an alpha pinene, beta pinene, or dipentene; a resin based on rosin, such as gum rosin, tail oil rosin, and wood rosin; hydrogenated products and esters of the above resins.

Additionally, examples of a plastic material include: a paraffin-based oil for reducing viscosity; a naphthenic oil for improving tackiness; and an aromatic oil for reducing cohesion or adding color and scent.

An application amount of the adhesive material 50 is in a range between 10 g/m² and 100 g/m², preferably between 20 g/m² and 50 g/m². If the application amount is not more than 10 g/m², an adhesive force exhibited when the absorbent article 1 is attached to a clothing item is weak; therefore, detachment, displacement, and the like are more likely to occur. If the application amount is not less than 100 g/m², the adhesive force becomes excessively high, and a process of detaching the absorbent article 1 from the clothing item is made difficult; therefore, it is not preferable.

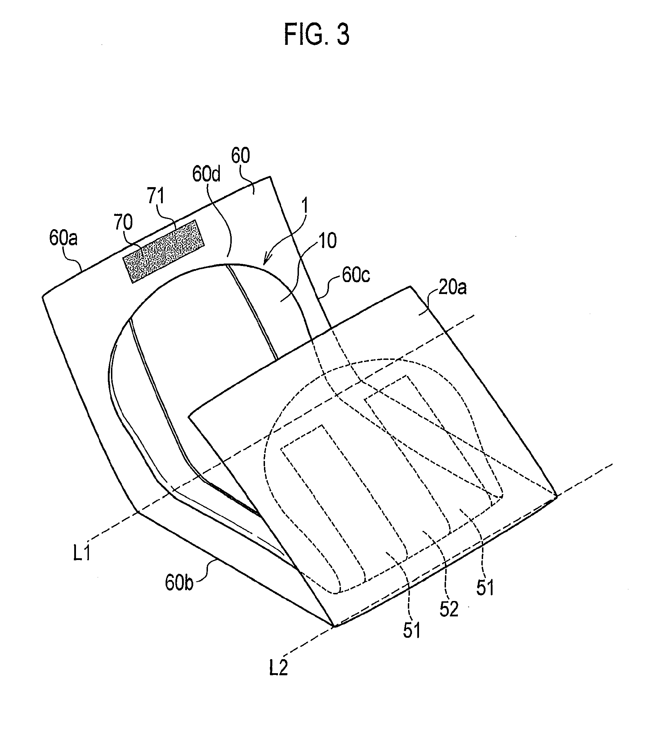

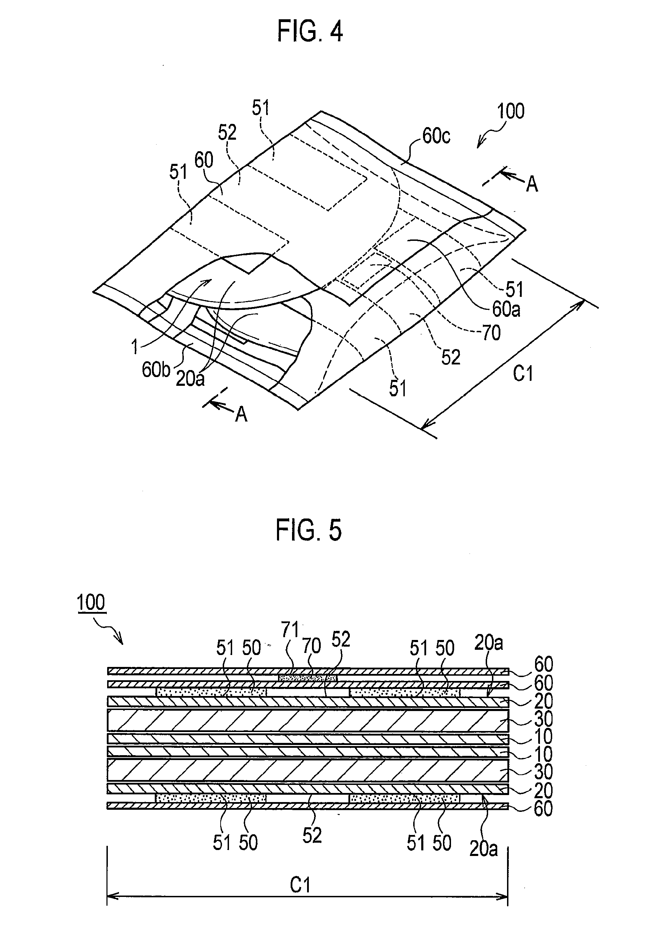

(Folding Structure of Wrapped Absorbent Article) FIG. 3 is a schematic view describing how the absorbent article 1 is folded and wrapped with a protection sheet 60. FIG. 4 is a perspective view showing a wrapped absorbent article 100 with a part of the wrapped absorbent article 100 cut away. FIG. 5 is a cross-sectional view taken along an A-A line in the vicinity of a central region C1 of the wrapped absorbent article 100 shown in FIG. 4. Note that the A-A line here is a hypothetical line passing through a second adhesive region 71 which is located in an end portion 60a of a later described protection sheet 60 and is provided with an adhesive material 70.

The wrapped absorbent article 100 includes the absorbent article 1 and the protection sheet 60, and is obtained by wrapping the absorbent article 1 with the protection sheet 60. The protection sheet 60 has a facing surface which faces the absorbent article 1 at the backsheet side thereof. In the protection sheet 60, the whole area of the facing surface (referred to as a treated surface 60d) is treated with a process which makes an adhesive material separable from the protection sheet 60. Further, another surface of the protection sheet 60 is also treated likewise with a process which makes an adhesive material thus separable. A film made of polypropylene, polyester, polyethylene, or polyvinyl alcohol, or a film made of a composite material formed by combining any multiple ones of the above materials is usable as the protection sheet 60. It is preferable that the thickness of the protection sheet 60 be between 5 µm and 50 µm.

Examples of a method for the process which makes an adhesive material separable includes: a method in which a separating agent is applied on the entire surface of the treated surface 60d, and then heated and dried; and a method in which a thin film is formed by spraying a separating agent onto the entire surface of the treated surface 60d. Here, examples of the separating agent include a silicone-based resin, a fluorine-based resin, and an octadecyl isocyanate-based resin. Additionally, the treated surface 60d may be treated with a process which forms microscopic undulations thereon. By reducing a contacting area between the treated surface 60d and the adhesive material, the detachment is facilitated.

The absorbent article 1 is folded at predetermined folding positions L1 and L2 with the topsheet 10 inside. The second adhesive region 71 coated with the adhesive material 70 is formed in a predetermined position which is located in the end portion 60a of the protection sheet 60 and faces the non-skin-surface 20a of the absorbent article 1. The protection sheet 60 is attached to a part of the protection sheet 60 by means of the adhesive material 70.

The adhesive material 70 is evenly applied on a surface of the second adhesive region 71. The adhesive material 70 is a hot-melt adhesive material made mainly of a rubber-based elastomer, a polyester-based elastomer, or a thermoplastic resin. The adhesive material 70 may be the same as the adhesive material 50.

The protection sheet 60 and the absorbent article 1 are bent with the topsheet 10 inside, whereby the second adhesive region 71 overlaps a region corresponding to the non-adhesive region 52. A part of the protection sheet 60 is folded back in such a manner as to reach a position where the part overlaps other part, which comes in contact with the non-skin-surface 20a of the absorbent article 1, of the protection sheet 60 (refer to FIG. 4). The end portion 60a of the protection sheet 60 is bonded, while being folded back, to the protection sheet 60 by means of the adhesive material 70. Edge portions 60b and 60c on both sides of the protection sheet 60 in a longitudinal direction of the protection sheet 60 are bonded together through thermal fusion bonding while being folded. The wrapped absorbent article 100 is formed in the manner described above.

FIG. 5 shows a cross-sectional view taken along the A-A line of the wrapped absorbent article 100. In this embodiment, the second adhesive region 71 coated with the adhesive material 70 is formed in the predetermined position which is located on the protection sheet 60 and faces the non-skin-surface 20a of the backsheet 20. The protection sheet 60 at the second adhesive region 71 is attached the region in the non-adhesive region 52 of the protection sheet 60 onto a region which corresponds to.

As described above, in the wrapped absorbent article 100, the second adhesive region 71 formed in the predetermined position on the protection sheet 60 exists in the position corresponding to the non-adhesive region 52 formed on the non-skin-surface 20a of the backsheet 20. Accordingly, even if the adhesive material 70 applied on the second adhesive region 71 permeates the protection sheet 60, dissolution of the adhesive material 70 with the adhesive material 50 applied on the non-skin-surface 20a of the backsheet 20 is avoidable. Furthermore, even if the adhesive material 50 permeates the protection sheet 60, dissolution of the adhesive material 50 with the adhesive material 70 applied on the second adhesive region 71 is avoidable.

Thereby, generation of an adhesive force stronger than expected is preventable between the adhesive material 70, which fixes the protection sheet 60, and the protection sheet 60. Accordingly, troubles, such as breaking of the protection sheet 60, are preventable when the wrapped absorbent article 100 is unwrapped. Regardless of a storage period and temperature changes in a storage environment, a user is enabled to easily unwrap the wrapped absorbent article 100 at all times.

Furthermore, the non-adhesive region 52 is formed on a central portion, which corresponds to the region S of the topsheet 10, of the backsheet 20, extending continuously in the longitudinal direction of the absorbent article 1. In such absorbent article 1, the central region of the absorbent article 1 is not attached to the clothing item, whereby the central of the absorbent article 1 is likely detached from the clothing item. Then, the region S of the topsheet 10 of the absorbent article 1 is likely to be lifted up toward the skin. Accordingly, a fitting property of the absorbent article 1 to the skin (vaginal opening) is improved.

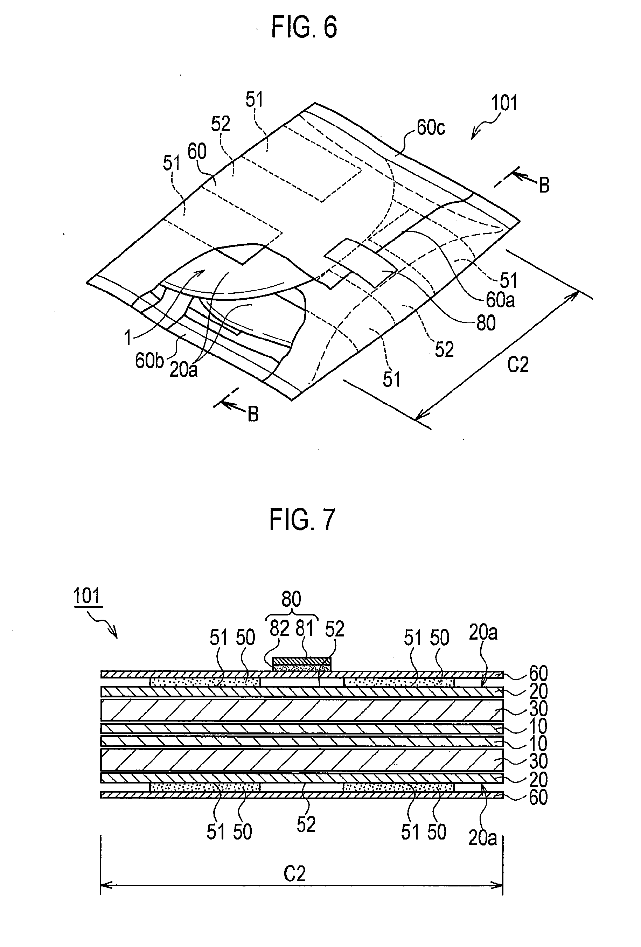

(Modified Examples of Protection Sheet Attaching Method) FIG. 6 is a perspective view showing a wrapped absorbent article 101, with a part of the wrapped absorbent article 101 cut away, shown as a modified example of the present embodiment. FIG. 7 is a cross-sectional view taken along a B-B line in the vicinity of a central region C2 of the wrapped absorbent article 101 shown in FIG. 6. The same constituent elements as those of the above described wrapped absorbent article 100 are denoted by the same reference numerals thereof, and their detailed description is omitted. The B-B line here is a hypothetical line passing through a later described position which is located on the protection sheet 60 beneath the end portion 60a of the protection sheet 60 and is to be attached by the adhesive tape 80.

The wrapped absorbent article 101 includes the adhesive tape 80. In the wrapped absorbent article 101, the end portion 60a of the protection sheet 60 is attached to a part of the protection sheet 60 by means of the adhesive tape 80. The adhesive tape 80 is formed of a film 81 provided as a base material and an adhesive material 82. Examples of the film 81 include single-layer films, such as a polypropylene film and a polyethylene film. Alternatively, the film 81 may be a laminated multilayer film formed by laminating multiple resin films. The thickness of the film 81 is in a range between 10 µm and 100 µm, preferably between 40 µm and 60 µm.

The adhesive material 82 is evenly applied on a surface of the film 81. The adhesive material 82 is a hot-melt adhesive material made mainly of a rubber-based elastomer, a polyester-based elastomer, or a thermoplastic resin. The adhesive material 82 may be the same as any of the adhesive materials 50 and 70.

In this modified example, the adhesive tape 80 is attached to a position corresponding to the non-adhesive region 52 formed on the backsheet 20. Accordingly, even if the adhesive material 82 permeates the protection sheet 60, dissolution of the adhesive material 82 with the adhesive material 50 applied on the non-skin-surface 20a of the backsheet 20 is avoidable. Furthermore, even if the adhesive material 50 permeates the protection sheet 60, dissolution of the adhesive material 50 and the adhesive material 82 applied on the adhesive tape 80 is avoidable.

Thereby, generation of an adhesive force stronger than expected is preventable between the adhesive material 82 of the adhesive tape 80, which fixes the protection sheet 60, and the protection sheet 60. Accordingly, troubles, such as breaking of the protection sheet 60, are preventable when the wrapped absorbent article 101 is unwrapped. Regardless of a storage period and temperature changes in a storage environment, a user is enabled to easily unwrap the wrapped absorbent article 101 at all times.

### (Modified Example 1)

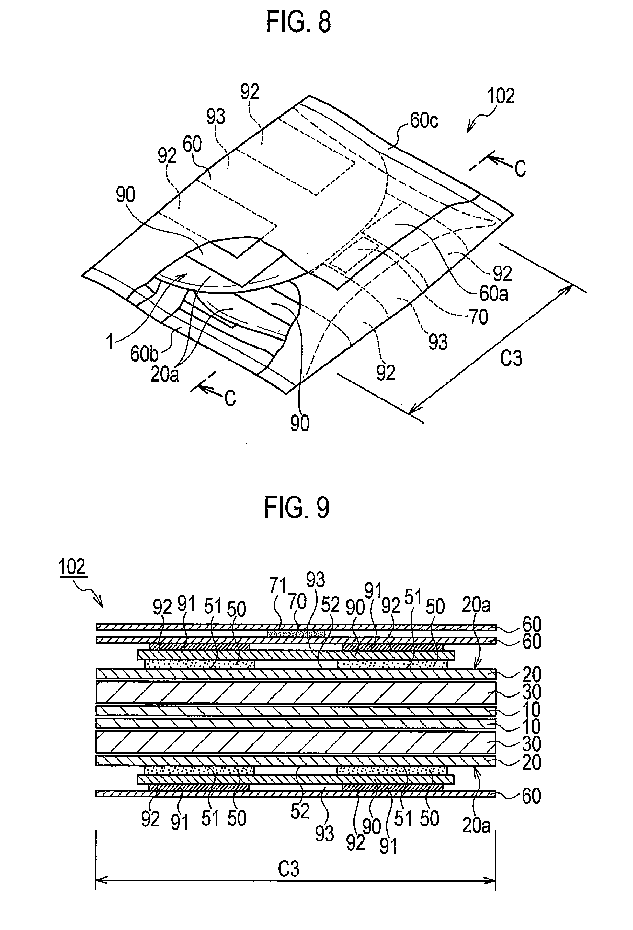

FIG. 8 is a perspective view showing a wrapped absorbent article 102, with a part of the wrapped absorbent article 102 cut away, shown as a modified example of this embodiment. FIG. 9 is a cross-sectional view taken along a C-C line in the vicinity of a central region C3 of the wrapped absorbent article 102 shown in FIG. 8. The same constituent elements as those of the above described wrapped absorbent articles 100 and 101 are denoted by the same reference numerals thereof, and their detailed description is omitted. Note that the C-C line here is a hypothetical line passing through a later described second adhesive region 71 which is located in the end portion 60a of the protection sheet 60 and is provided thereon with the adhesive material 70.

The wrapped absorbent article 102 includes a release sheet 90. The release sheet 90 is disposed between the protection sheet 60 and the backsheet 20. The release sheet 90 is a sheet having one surface treated with a process which makes an adhesive material separable from the surface.

In the wrapped absorbent article 102, the second adhesive region 71, coated with the adhesive material 70, is formed in a predetermined position being located in the end portion 60a of the protection sheet 60 and facing the non-skin-surface 20a of the absorbent article 1. The protection sheet 60 is attached to a part of the protection sheet 60 by means of the adhesive material 70. The protection sheet 60 and the release sheet 90 are bonded and fixed to each other by means of an adhesive material 91, such as a mot-melt adhesive material. On a non-skin-surface of the release sheet 90, release-sheet side adhesive regions 92 coated with the adhesive material 91 and a release-sheet side non-adhesive region 93 coated with the adhesive material 91 are formed. The protection sheet 60 at the second adhesive region 71 is attached to a region in the release-sheet side non-adhesive region 93.

As described above, in the wrapped absorbent article 102, the second adhesive region 71 formed in the predetermined position in the end portion 60a on the protection sheet 60 exists in a position corresponding to the release-sheet side non-adhesive region 93 formed on the non-skin-surface of the release sheet 90. Accordingly, even if the adhesive material 70 applied on the second adhesive region 71 permeates the protection sheet 60, dissolution of the adhesive material 70 with the adhesive material 91 applied on the release sheet 90 is avoidable. Furthermore, even if the adhesive material 91 permeates the protection sheet 60, dissolution of the adhesive material 91 with the adhesive material 70 applied on the second adhesive region 71 is avoidable.

Thereby, generation of an adhesive force stronger than expected is preventable between the adhesive material 70, which fixes the protection sheet 60, and the protection sheet 60. Accordingly, troubles, such as breaking of the protection sheet 60, are preventable when the wrapped absorbent article 102 is unwrapped. Regardless of a storage period and temperature changes in a storage environment, a user is enabled to easily unwrap the wrapped article 102 at all times.

### (Modified Example 2)

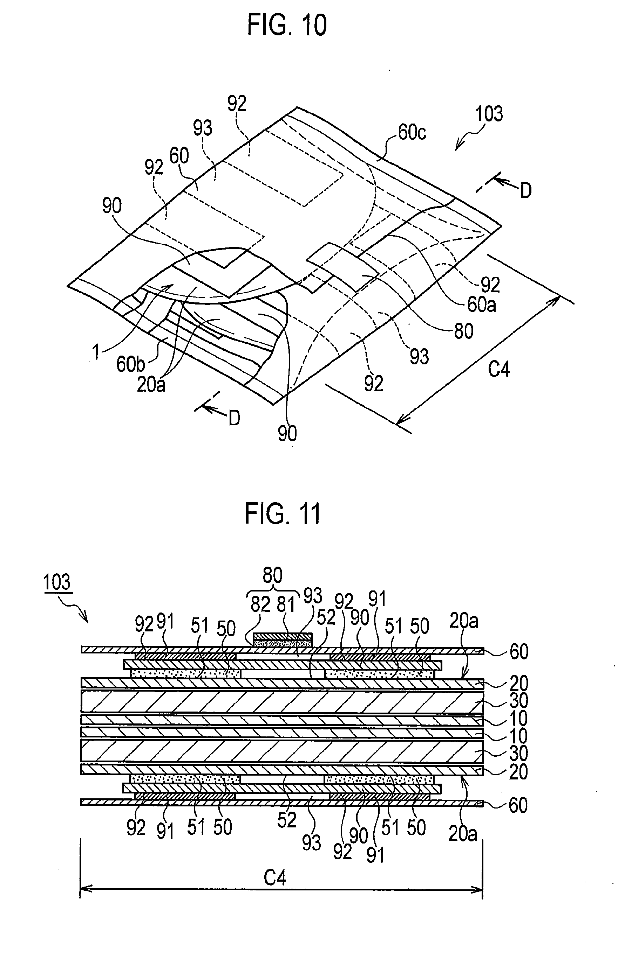

FIG. 10 is a perspective view showing a wrapped absorbent article 103, with a part of the wrapped absorbent article 103 cut away, shown as a modified example of this embodiment. FIG. 11 is a cross-sectional view taken along a D-D line in the vicinity of a central region C4 of the wrapped absorbent article 103 shown in FIG. 10. The same constituent elements as those of the above described wrapped absorbent articles 100, 101 and 102 are denoted by the same reference numerals thereof, and their detailed description is omitted. Note that the D-D line here is a hypothetical line passing through a later described position which is located on the protection sheet 60 beneath the end portion 60a of the protection sheet 60 and is to be attached by the adhesive tape 80.

The wrapped absorbent article 103 includes the release sheet 90. In the wrapped absorbent article 103, the end portion 60a of the protection sheet 60 is attached to a part of the protection sheet 60 by means of the adhesive tape 80. The protection sheet 60 and the release sheet 90 is bonded and fixed to each other by means of the adhesive material 91, such as a mot-melt adhesive material.

On a non-skin-surface of the release sheet 90, the release sheet side adhesive regions 92 coated with the adhesive material 91 and the release sheet side non-adhesive region 93 not coated with the adhesive material 91 are formed. As for the protection sheet 60 for the wrapped absorbent article 103 including the release sheet 90, a surface of the protection sheet 60 does not need to be treated with the process which makes an adhesive agent separable from the surface.

In this modified example, the adhesive tape 80 is attached to a position corresponding to the release sheet side non-adhesive region 93. Accordingly, even if the adhesive material 82 permeates the protection sheet 60, dissolution of the adhesive material 82 with the adhesive material 91 is avoidable. Furthermore, even if the adhesive material 91 permeates the protection sheet 60, dissolution of the adhesive material 91 with the adhesive material 82 applied on the second adhesive region 70 is avoidable.

Thereby, generation of an adhesive force stronger than expected is preventable between the adhesive tape 80, which fixes the protection sheet 60, and the release sheet 90. Furthermore, generation of an adhesive force stronger than expected is preventable between the release sheet 90 and the protection sheet 60. Accordingly, troubles, such as breaking of the protection sheet 60, are preventable when the wrapped absorbent article 103 is unwrapped. Regardless of a storage period and temperature changes in a storage environment, a user is enabled to easily unwrap the wrapped absorbent article 103 at all times.

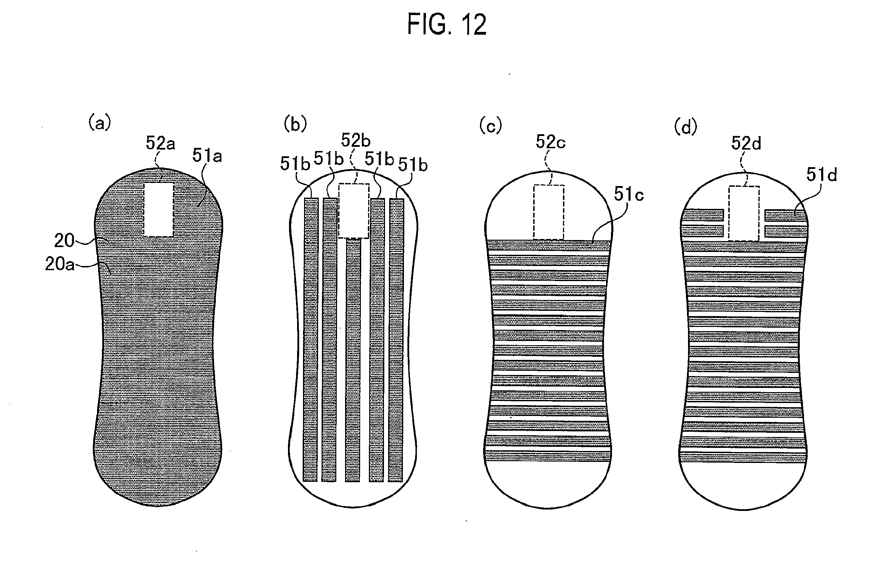

(Modified examples of Non-adhesive Region Formed on Backsheet) FIGS. 12 (a) to 12 (d) are plan views, each explaining a modified example of the first adhesive regions and the non-adhesive region formed on the backsheet 20, of a backsheet side of an absorbent article.

In a modified example shown in FIG. 12 (a), a first adhesive region 51a is formed in the almost whole area of the backsheet 20, and a non-adhesive region 52a is formed only in a region corresponding to a second adhesive region formed in the end portion 60a of the protection sheet 60.

In a modified example shown in FIG. 12 (b), first adhesive regions 51b are respectively formed to extend continuously in the longitudinal direction of the absorbent article 1, and form multiple lines. A non-adhesive region 52b is formed in a region corresponding to the second adhesive region formed on the end portion 60a of the protection sheet 60. Therefore, the first adhesive region 51b that is formed in the center of the multiple adhesive regions 51 is shorter than the other ones.

In a modified example shown in FIG. 12 (c), first adhesive regions 51c are formed in a location in the vicinity of a central region of the backsheet 20, excluding both end portions of the backsheet 20. The first adhesive regions 51c are formed along a width direction of the absorbent article 1. Substantially, a non-adhesive region 52a is formed by not applying adhesive material 50 to the both end portions. These configurations of the first adhesive regions 51c and the non-adhesive region 52c are highly effective on manufacture of the absorbent article 1 in a case where the absorbent article 1 is conveyed along a longitudinal direction of the absorbent article 1 in the manufacturing processes.

In a modified example shown in FIG. 12 (d), first adhesive regions 51d are formed along a width direction of the absorbent article 1. In the modified example shown in FIG. 12 (d), in order to avoid insufficient adherence to a clothing item in the both end portions of the absorbent article 1, the first adhesive regions 51d are also applied to both portions located next to the non-adhesive region 52d in a width direction of the absorbent article 1.

### (Other embodiment)

As described above, the details of the present embodiment have been disclosed by using the embodiment. However, it should not be understood that the description and drawings which constitute part of this disclosure limit the present embodiment. From this disclosure, various alternative embodiments, examples, and operation techniques will be easily found by those skilled in the art.

The embodiment may be modified as follows. Although the absorbent article has been described as a sanitary napkin in the above described embodiment, the present embodiment may also be applied to a so-called panty liner, an incontinence product (called an incontinent pad), and the like.

As for the absorbent article, a wing portion may be formed outside of a central region of the absorbent article in a width direction the absorbent article. Additionally, the absorbent article may be provided with a leakage preventing portion (so-called gathering) which prevents body fluid from leaking outward in a width direction of the absorbent article. The wing portion may be, for example, folded back onto a topsheet. The absorbent article is folded at predetermined folding positions with the topsheet inside while the wing portion is thus folded in.

In this embodiment, the structure in which the absorbent article is folded at two predetermined folding positions (L1 and L2) has been described. However, the number of times an absorbent article is folded is not limited. For example, the absorbent article may be folded atone folding position, or may be folded at two or more positions. Moreover, the absorbent article may be rolled up in a longitudinal direction the absorbent article (that is, has no folding position).

In the present embodiment, the adhesive material 70 applied on the protection sheet 60 and the adhesive material 91 applied on the release sheet 90 only need to be disposed so as not to overlap with each other with the protection sheet 60 therebetween. Therefore, regions coated with the adhesive material 70 and the adhesive material 91 are not limited to those in the examples shown in FIGS. 8 to 11. Furthermore, if the adhesive material 70, the adhesive material 50 applied on the backsheet 20, and the adhesive material 91 applied on the release sheet 90 are disposed so as not to overlap with each other with the protection sheet 60 and the release sheet 90 therebetween, dissolution of these adhesive materials is less likely to occur when the absorbent article is unwrapped. Note that the release sheet 90 only needs to be in a size which allows the release sheet 90 to cover the adhesive material 50 applied on the first adhesive region 51, and the size is not limited to those shown in the embodiment.

As described above, the present embodiment naturally includes various embodiments which are not described herein. Accordingly, the technical scope of the present embodiment should be determined only by the technical feature in the scope of claims regarded as appropriate based on the description.

### [Industrial Applicability]

According to the present embodiments, it is possible to provide the wrapped absorbent article enables easy unwrapping while preventing a trouble at unwrapping.

## Claims

1. A wrapped absorbent article, comprising:
an absorbent article including
a top sheet including a contact region which comes in contact with an excretory part of a wearer,
a backsheet having liquid impermeability which allows no permeation of liquid, and including a first adhesive material which is provided on a non-skin-surface of the backsheet to be attached to a clothing item, and
an absorber disposed between the topsheet and the backsheet; and
a protection sheet having a facing surface which faces the absorbent article on the backsheet side while keeping the first adhesive material separable, wherein
the absorbent article is wrapped with the protection sheet in a state where the non-skin-surface of the absorbent article and the facing surface of the protection sheet are in contact with each other,
the non-skin-surface of the backsheet is provided with a first adhesive region coated with the first adhesive material and a non-adhesive region not coated with the first adhesive material,
the protection sheet includes a second adhesive region coated with a second adhesive material,
the absorbent article and the protection sheet are bent with the topsheet inside so that the second adhesive region overlaps a region included in the non-adhesive region; and
the protection sheet is attached to the region included in the non-adhesive region of the protection sheet at the second adhesive region.

2. The wrapped absorbent article according to claim 1, wherein the non-adhesive region is formed in a central region of the absorbent article in a width direction the absorbent article to extend continuously in a longitudinal direction of the absorbent article.

3. The wrapped absorbent article according to any one of claims 1 and 2, further comprising an adhesive tape provided on an end portion of the protection sheet, wherein:
the adhesive tape includes the second adhesive region coated with the second adhesive material on a surface of the adhesive tape, and
the adhesive tape is attached to a position included in the non-adhesive region.

4. A wrapped absorbent article comprising:
an absorbent article including
a top sheet including a contact region which comes in contact with an excretory part of a wearer,
a backsheet having liquid impermeability which allows no permeation of liquid, and provided with a first adhesive material on a non-skin-surface to be attached to a clothing item, and
an absorber disposed between the topsheet and the backsheet;
a release sheet having a facing surface which faces the absorbent article on the backsheet side while keeping the first adhesive material separable; and
a protection sheet disposed on a non-skin-surface of the release sheet, wherein
the absorbent article is wrapped with the protection sheet in a state where the non-skin-surface of the absorbent article and the facing surface of the release sheet are in contact with each other,
the non-skin-surface of the release sheet is provided with a release sheet side first adhesive region coated with a release sheet adhesive material bond together the release sheet and the protection sheet, and with a release sheet side non-adhesive region not coated with the release sheet adhesive material,
the protection sheet includes a second adhesive region coated with a second adhesive material,
the absorbent article and the protection sheet are bent with the topsheet inside so that the second adhesive region overlaps a region included in the release sheet side non-adhesive region, and
the protection sheet is attached to the region included in the non-adhesive region of the protection sheet at the second adhesive region.
